# DEMANDE DE BREVET EUROPEEN

(11) **EP 1 269 984 A1**
(43) Date de publication de la demande: **02.01.2003**
(21) Numéro de dépôt: 02291333.9
(22) Date de dépôt: 30.05.2002
(51) Int. Cl.: A61K 7/48, A61K 31/352, A61P 17/00

(54) **Composition cosmétique et/ou dermatologique et/ou pharmaceutique à application topique, contenant au moins un composé inhibiteur de l'enzyme 3beta-HSD**

(30) Priorité: 30.05.2001 FR 0107102
(71) Demandeur: L'OREAL, 75008 Paris (FR)
(72) Inventeur: Breton, Lionel, 78000 Versailles (FR)
(74) Mandataire: Catherine, Alain

(57) **Abrégé**

L'invention se rapporte à une composition cosmétique et/ou dermatologique et/ou pharmaceutique à application topique ou par administration orale, caractérisée en ce qu'elle contient une quantité efficace d'au moins un composé inhibiteur de l'enzyme 3β-hydroxystéroïde déhydrogénase. Cette composition est destinée au traitement de certains effets du vieillissement chronologique et du photo-vieillissement de la peau.

## Description

L'invention se rapporte à une composition cosmétique et/ou dermatologique et/ou pharmaceutique à application topique ou administration orale, contenant une quantité efficace d'au moins un composé inhibiteur de l'enzyme 3β-hydroxystéroïde déhydrogénase (3β-HSD). Cette composition est destinée au traitement de certains signes du vieillissement cutané, endogène et/ou exogène.

Le vieillissement cutané résulte des effets sur la peau de facteurs intrinsèques et extrinsèques. Cliniquement, les signes du vieillissement se traduisent par l'apparition de rides et ridules, par un relâchement des tissus cutanés et sous-cutanés, par une perte de l'élasticité cutanée, par une atonie de la texture de la peau, et par le jaunissement de la peau qui devient plus terne et sans éclat.

Sur les zones de la peau qui ont été exposées au soleil tout au long de la vie, essentiellement le visage, le décolleté, les mains et les avant bras, on observe souvent des taches pigmentaires, des télangiectasies et une élastose.

Certains de ces signes sont plus particulièrement liés au vieillissement intrinsèque ou physiologique, c'est-à-dire au vieillissement lié à l'âge. Le vieillissement intrinsèque est encore appelé vieillissement chronologique.

D'autres signes sont plus spécifiques du vieillissement extrinsèque, c'est-à-dire du vieillissement provoqué d'une manière générale par l'environnement ; il s'agit plus particulièrement du photo-vieillissement dû à l'exposition au soleil, à la lumière ou à tout autre rayonnement.

Les changements de la peau résultant du vieillissement chronologique sont la conséquence d'une sénescence génétiquement programmée où interviennent des facteurs endogènes. Ce vieillissement intrinsèque provoque notamment un ralentissement du renouvellement des cellules de la peau. Histologiquement, la peau est globalement amincie, tant au niveau épidermique que dermique. En outre, on observe un relâchement des tissus cutanés et sous-cutanés, qui se traduit par une texture de peau atone, un relâchement du microrelief cutané, une fermeté cutanée diminuée, et une peau globalement flasque. La densité des macromolécules fibreuses du derme (élastine et collagène) est diminuée. On peut également observer, chez certaines personnes, une diminution du taux de DHEA endogène.

Le vieillissement extrinsèque entraîne, en ce qui le concerne, des changements histopathologiques tels que l'accumulation excessive de matière élastique non fonctionnelle dans le derme supérieur et la dégénérescence des fibres de collagène. Ces changements histopathologiques conduisent également à une altération du derme et à une diminution de la fermeté et de l'élasticité de la peau.

Dans les deux cas de vieillissement chronologique ou extrinsèque, ce sont notamment les fibres de collagène qui sont altérées. Ces fibres de collagène assurent l'élasticité du derme. Les fibres de collagène sont constituées de fibrilles scellées les unes aux autres, formant ainsi plus de 10 types de structures différentes. La solidité du derme est en grande partie due à l'enchevêtrement des fibres de collagène tassées les unes contre les autres en tous sens. Les fibres de collagène participent à l'élasticité et à la tonicité de la peau et/ou des muqueuses.

Les fibres de collagène sont constamment renouvelées mais ce renouvellement ralentit avec l'âge, ce qui entraîne l'amincissement du derme. Cet amincissement du derme est également dû à des causes pathologiques comme par exemple l'hypersécrétion d'hormones corticoïdes, certaines pathologies ou encore des carences vitaminiques (cas de la vitamine C dans le scorbut). Il est également admis que les facteurs extrinsèques comme les rayons ultraviolets, le tabac ou certains traitements (glucocorticoïdes, Vitamine D et dérivés par exemple) ont également un effet sur la peau et sur son taux de collagène.

L'invention s'intéresse particulièrement à la correction des signes du vieillissement chronologique. Cette invention porte sur l'activation du renouvellement cellulaire et/ou sur l'amélioration de la densité des macromolécules fibreuses du derme. Cette invention porte également sur le maintien du taux de DHEA endogène dans la peau chez des sujets pour lesquels celui-ci a tendance a être diminué.

L'invention porte également sur la correction des rides et ridules de la peau, sur la correction du relâchement des tissus cutanés et sous-cutanés et sur l'amélioration de l'éclat de la peau.

A l'heure actuelle, il existe de nombreuses compositions qui prétendent traiter les rides et les ridules de la peau ou raffermir les tissus cutanés, mais ces compositions ne traitent qu'incomplètement et temporairement ces désordres morphologiques.

Des compositions ont certes déjà été proposées pour traiter plus efficacement et à long terme les rides et les ridules, raffermir les tissus cutanés et donner de l'éclat à une peau atone, l'apport exogène d'hormones stéroïdes, et en particulier sur l'apport de déhydroépi-androstérone (DHEA) et/ou de ses dérivés (comme par exemple le sulfate de déhydroépi-androstérone ou S-DHEA) dans des compositions cosmétiques, et/ou pharmaceutiques, et/ou dermatologiques permettent de donner des résultats satisfaisants dans le traitement de ces altérations du tissu cutané.

Par apport exogène d'hormones stéroïdes, on entend au sens de la demande, tout apport d'hormones stéroïdes provenant de l'extérieur de l'organisme mais qui pourraient être sécrétées par l'organisme.

Il existe de nombreux brevets décrivant des compositions cosmétiques ou dermatologiques à application topique comprenant de la déhydroépi-androstérone et/ou ses dérivés. Par exemple, le brevet américain US-5,989,568 décrit l'utilisation du sulfate de déhydroépi-androstérone dans une composition topique pour traiter les rides, les ridules et/ou lutter contre le relâchement cutané et/ou sous-cutané et/ou raviver l'éclat de la peau.

Cependant, l'inconvénient majeur de ces compositions consiste en ce qu'elles contiennent justement des hormones stéroïdes qui sont soumises à des contraintes spécifiques d'utilisation et de réglementation.

Il existe donc aujourd'hui dans l'état de la technique un fort besoin de disposer d'une méthode permettant de traiter efficacement et à long terme certains signes du vieillissement cutané sans apport exogène d'hormones stéroïdes et notamment de DHEA. Personne à ce jour n'a imaginé réguler positivement le taux tissulaire de DHEA sans apport extérieur de cette molécule.

La présente invention vise justement à la satisfaction d'un tel besoin.

Or, la demanderesse a découvert de manière surprenante que l'enzyme 3β-hydroxystéroïde deshydrogénase (également appelé 3β HSD) est exprimé au niveau de l'épiderme et du derme des êtres humains, c'est-à-dire respectivement au niveau des kératinocytes épidermiques et des fibroblastes dermiques humains.

Par expression de l'enzyme 3β-HSD, on entend au sens de la demande l'expression de l'ARNm de la 3β-HSD qui après traduction conduit à la synthèse de la protéine : l'enzyme.

Il est connu que l'enzyme 3β-HSD est impliqué dans le catabolisme de la DHEA endogène en androstédione.

Une diminution de l'activité et/ou de l'expression de cet enzyme équivaut par conséquent à ralentir la dégradation de la DHEA et à maintenir un taux endogène de DHEA suffisant au niveau du derme et de l'épiderme sans apport exogène de DHEA.

La présente invention a donc pour objet une composition cosmétique et/ou dermatologique et/ou pharmaceutique à application topique ou par administration orale, caractérisée en ce qu'elle contient une quantité efficace d'au moins un composé inhibiteur de l'enzyme 3β-hydroxystéroïde déhydrogénase.

L'application topique d'une telle composition permet de réguler positivement le taux endogène cutané de DHEA et/ou de ses dérivés (tel que notamment le sulfate de DHEA) et le maintenir à un taux physiologique ou supraphysiologique.

La composition contient un milieu cosmétiquement ou dermatologiquement acceptable, c'est-à-dire compatible avec les tissus cutanés. Ainsi, la composition peut être appliquée sur tout le corps humain.

Le composé inhibiteur 3β-HSD selon l'invention est présent dans la composition à raison de 0,0000001 à 25% en poids, et de préférence de 0,00001 à 10% en poids par rapport au poids total de la composition.

De manière avantageuse, le composé inhibiteur de 3β-HSD utilisable selon l'invention peut être choisi parmi les isoflavones et ses dérivés, et de préférence parmi la daidzéine et la génistéine, ainsi que leurs dérivés isoflavanes : respectivement equol ou 4',7-dihydroxy-isoflavane, et 5-hydroxyequol ou 4',5,7-trihydroxy-isoflavane.

Une isoflavone préférée selon l'invention est la génistéine et/ou son équivalent flavane : 5-hydroxyequol ou 4',5,7 trihydroxy-isoflavane.

Sont également revendiqués comme composés inhibiteurs de 3β-HSD selon l'invention, les composés choisis parmi l'Epostane (WIN 32729), le Trilostane (WIN 24540), le 11-α-bromoacetoxy-progestérone, des dérivés 11-alkyl de la progestérone, des dérivés 3 ou 4 cétostéroïdes, le RU486, et le N,N-diméthyl-4-méthyl-3-oxo-4-aza-5-androstane-17-β-carboxamide (4-MA) ; le cholestérol, ses esters et ses dérivés, la 16,α-hydroxydehydroepiandrosterone, le 17-β-oestradiol, l'éthynylestradiol, l'acétate de chloramidone, le cyanotriméthylandrostenolone.

Bien entendu, il est possible d'associer le ou les composés inhibiteurs de 3β-HSD selon l'invention à des hormones naturelles ou synthétiques choisies parmi les hormones oestrogéniques, progestatives et androgéniques, comme la progestérone, la testostérone, le broparestrol, l'oestrone, l'acétate de prégnénolone, la prégnénolone, la 17 β-hydroxyprogestérone, le propionate de testostérone, l'androstènedione et les androstanediols.

Il est également possible d'associer le composé inhibiteur de 3β-HSD selon l'invention à des molécules et/ou des extraits végétaux mimétiques de l'oestradiol et/ou de la progestérone.

Par extrait végétal mimétique, on entend, au sens de la demande, tout extrait végétal capable de mimer l'action de l'oestradiol et/ou de la progestérone pour corriger les effets du vieillissement de la peau, sans les inconvénients liés à l'emploi des hormones.

Enfin, il est encore possible d'associer le ou les inhibiteurs de 3β-HSD, et plus particulièrement le ou les isoflavones selon l'invention, à de la déhydroépi-androstérone (DHEA) et/ou des dérivés de DHEA, et notamment du sulfate de DHEA, connus pour présenter des propriétés antivieillissement, et plus particulièrement présentant une activité marquée dans les domaines du traitement des rides et/ou des ridules, de la lutte contre le relâchement cutané et/ou sous-cutané et/ou du ravivage de l'éclat.

Au titre de l'invention, il est également possible d'associer le ou les inhibiteurs de 3β-HSD à des dérivés de DHEA tels que la 7OH-DHEA, la 7 Ceto-DHEA, et la 3β-acétoxy-7-oxo-DHEA.

Les compositions utilisables dans l'invention peuvent se présenter sous toutes les formes galéniques classiquement utilisées pour une application topique et notamment sous forme d'une solution ou suspension aqueuse, alcoolique ou hydroalcoolique ou d'une suspension huileuse ou d'une solution ou d'une dispersion du type lotion ou sérum, d'une émulsion de consistance liquide ou semi-liquide du type lait, obtenues par dispersion d'une phase grasse dans une phase aqueuse (H/E) ou inversement (E/H), ou d'une suspension ou émulsion de consistance molle de type crème (H/E) ou (E/H), ou d'un gel aqueux ou anhydre, d'un onguent, d'une poudre libre ou compactée à utiliser telle quelle ou à incorporer dans un excipient, ou sous forme de microcapsules ou microparticules, ou de dispersions vésiculaires de type ionique et/ou non ionique.

Les compositions utilisées dans le procédé selon l'invention peuvent comporter au moins une matière grasse liquide ou solide.

Comme matières grasses selon l'invention, on peut citer les huiles minérales (huile de vaseline), les huiles végétales (fraction liquide du beurre de karité, huile de tournesol), les huiles animales (perthydrosqualène), les huiles de synthèse (huile de Purcellin), les huiles siliconées (cyclométhicone), et les huiles fluorées (perfluoropolyéthers). On peut aussi utiliser comme matières grasses des alcools gras, des acides (acide stéarique), des cires (paraffine, carnauba, cire d'abeilles).

Les huiles, les émulsionnants et les coémulsionnants utilisés dans la composition sous forme d'émulsion sont choisis parmi ceux classiquement utilisés dans le domaine cosmétique ou dermatologique.

Comme émulsionnants utilisables dans l'invention, on peut citer par exemple le stéarate de glycérol, le polysorbate 60 et le mélange de PEG-6/PEG632/Glycol Stéarate vendu sous la dénomination de Tefose® 63 par la société Gattefosse.

L'émulsionnant et le coémulsionnant sont présents, dans la composition, en une proportion allant de 0,3% à 30% en poids, et de préférence de 0,5 à 20% en poids par rapport au poids total de la composition.

De façon connue, la composition selon l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes.

Les quantités de ces différents adjuvants sont celles classiquement utilisées dans le domaine considéré, par exemple de 0,001% à 20% du poids total de la composition.

Ces adjuvants, selon leur nature, peuvent être introduits dans la phase grasse, dans la phase aqueuse et/ou dans les sphérules lipidiques.

Comme solvants utilisables selon l'invention, on peut citer les alcools inférieurs, notamment l'éthanol et l'isopropanol, le propylène glycol.

Comme gélifiants hydrophiles utilisables selon l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles.

Comme gélifiants lipophiles utilisables selon l'invention, on peut citer les argiles modifiées comme des bentones, les sels métalliques d'acides gras comme les stéarates d'aluminium et la silice hydrophobe, ou encore l'éthylcellulose et le polyéthylène.

Comme actifs lipophiles ou hydrophiles utilisables dans l'invention en vue de parfaire le traitement des rides, ridules, la lutte contre le relâchement cutané et/ou sous-cutané et/ou l'éclat de la peau, on peut citer par exemple les rétinoïdes (rétinol et ses esters, rétinal, acide rétinoïque et ses dérivés, rétinoïdes, notamment deux décrits dans les documents FR-A-2570377, EP-A-199636, EP-325540, EP-A-402072), les α-hydroxy-acides (glycolique, lactique, malique, citrique, tartrique, mandélique), les β-hydroxy-acides (acides salicyclique et ses dérivés notamment alcoylés), les α-céto-acides, les β-céto-acides, les peroxydes comme le peroxyde de benzoyle, les vitamines notamment E, F, les actifs anti-radicaux comme la superoxyde-dismutase, le sélénium, le zinc, les caroténoïdes, notamment béta-carotène, lycopène, lutéine, zeaxanthine.

L'invention se rapporte également à un procédé pour corriger les effets du vieillissement chronologique et/ou du photo-vieillissement de la peau, consistant à appliquer sur la peau la composition selon l'invention.

Plus particulièrement, l'invention se rapporte à un procédé pour activer le renouvellement cellulaire épidermique et/ou dermique et/ou améliorer la densité des macromolécules fibreuses du derme, et/ou réguler la synthèse et/ou la dégradation des protéines matricielles du derme, et/ou corriger le relâchement des tissus cutanés et sous-cutanés, et/ou raviver l'éclat de la peau et/ou diminuer les rides et/ou ridules, consistant à appliquer sur la peau la composition selon l'invention.

L'invention a aussi pour objet un procédé pour réguler les processus de pigmentation de la peau et des phanères, et/ou de la séborrhée et/ou de la croissance du cheveu et/ou des poils et/ou des ongles, consistant à appliquer sur la peau et/ou les phanères la composition selon l'invention.

Enfin, l'invention concerne également l'utilisation dans une composition cosmétique et/ou dermatologique et/ou pharmaceutique à application topique, d'une quantité efficace d'au moins un composé inhibiteur de l'enzyme 3β-hydroxystéroïde déhydrogénase comme agent correcteur des effets du vieillissement chronologique et/ou du photo-vieillissement de la peau, et plus particulièrement comme :
- agent d'activation du renouvellement cellulaire et/ou agent d'amélioration de la densité des macromolécules fibreuses du derme, et/ou
- agent régulateur de la synthèse et/ou de la dégradation des protéines matricielles du derme, et/ou
- agent de correction du relâchement des tissus cutanés et sous-cutanés, et/ou
- agent ravivant l'éclat de la peau et/ou de réduction des rides et ridules de la peau, et/ou
- agent de régulation des processus de pigmentation de la peau et des phanères et/ou de la séborrhée et/ou de la croissance du cheveu et/ou des poils et/ou des ongles.

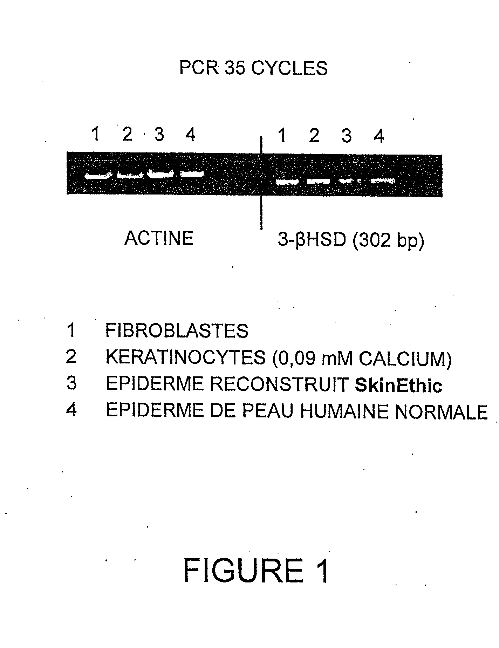

La figure 1 représente les résultats d'une électrophorèse en gel d'aragose après Reverse-Transcription sur ARN de différents échantillons cellulaires issus de peaux normales ou reconstruites *in vitro.*

Les exemples suivants illustrent l'invention. Dans ces exemples, les proportions indiquées sont des pourcentages en poids.

### EXEMPLES

### EXEMPLE 1

### Mise en évidence de l'expression de l'ARNm de la 3β-HSD au niveau de l'épiderme et du derme

On souhaite vérifier l'expression de l'enzyme 3β-hydroxystéroïde deshydrogénase (3β-HSD) dans différents modèles cellulaires dérivés de peau humaine : culture cellulaire de kératinocytes, culture cellulaire de fibroblastes, peaux reconstruites ou prélèvements chirurgicaux de peau humaine.

Pour cela, on va rechercher l'expression de l'ARNm de la 3β-HSD par la méthode de RT-PCR. La RT-PCR est une méthode qui permet de détecter la présence, même faible, de l'ARNm codant pour la protéine recherchée (en l'occurrence, 3β-HSD).

### Mode opératoire

### 1. Préparations d'ARN totaux à partir de 4 origines différentes (culture cellulaire de kératinocytes, culture cellulaire de fibroblastes, peaux reconstruites ou prélèvements chirurgicaux de peau humaine).

1. ARN total de fibroblastes dermiques humains normaux (NHDF) cultivés en monochouche en milieu MEM/M199 supplémenté par 1% de sérum de veau foetal.
2. ARN total de kératinocytes épidermiques humains normaux (NHEK) cultivés en monocouches en milieu SFM (Gibco) sans complémentation en calcium (milieu pauvre en calcium, 0,09 mM Ca²+).
3. ARN total d'épiderme reconstruit SkinEthic (Laboratoires SkinEthic, Nice, France) âgé de 17 jours, cultivé en milieu SkinEthic.
4. ARN total d'épiderme cutané humain normal (plastie mammaire).

Les cellules et tissus congelés ont été lysées et les ARN totaux ont été extraits à l'aide de TriReagent (Sigma), selon le protocole préconisé par le fournisseur.

### 2. Analyse

- A partir des 4 conditions expérimentales (culture cellulaire de kératinocytes, culture cellulaire de fibroblastes, peaux reconstruites ou prélèvements chirurgicaux de peau humaine) les ARN totaux sont purifiés (extraction des ARN totaux).
- A partir de l'ARN total, l'ADN complémentaire (cDNA) des différents ARNm (présents dans l'ARN total purifié) est synthétisé par Reverse-Transcription avec une amorce oligo (dT).
- Une séquence spécifique (302 paires de base), correspondant à la séquence de la 3β-HSD, est amplifiée par PCR (Polymerase Chain Reaction) en utilisant des oligo-nucléotides spécifiques.
- Les fragments amplifiés sont ensuite analysés par électrophorèse en gel d'agarose (1,7%), en présence de bromure d'éthidium. La saisie des images a été réalisée sur GelPrint 2000i (BioPhotonics Corp).

### 3. Résultats

Un fragment unique correspondant à la taille du fragment de 302 paires de base attendu (qui correspond à la protéine 3β-HSD) a été clairement détecté dans tous les échantillons testés (cultures, peaux reconstruites ou prélèvements chirurgicaux).

Les résultats sont illustrés sur la figure 1.

L'ARNm de la 3β-HSD est donc exprimé à la fois au niveau de l'épiderme (kératinocytes) et au niveau du derme (fibroblastes).

Cette observation permet de conclure que la protéine 3β-HSD est vraisemblablement présente dans la peau.

### EXEMPLE 2

| **Composition 1 :** **Capsules molles /Administration par voie orale** | |
|---|---|
| *Excipients :* | |
| Huile de Soja | 40 mg |
| Huile de Germe de Blé | 85 mg |
| Lécithines de Soja | 25 mg |
| *Vitamine* / *antioxydants :* | |
| Tocopherols naturels | 3 mg |
| Vitamine C | 50 mg |
| Lycopène | 5 mg |
| *Composants :* | |
| Génistéine | 25 mg |

| **Composition 2 :** **Crème de soin (émulsion huile dans eau) pour activer le renouvellement cellulaire épidermique et/ou améliorer la densité des macromolécules fibreuses** | |
|---|---|
| Génistéine | 0,1 % |
| 7OH-DHEA | 0,2 % |
| Stéarate de glycérol | 2,00 % |
| Polysorbate 60 (Tween 60® vendu par la société ICI) | 1,00 % |
| Acide stéarique | 1,40 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100,00 % |

| **Composition 3 :** **Gel pour la peau destiné à réguler l'éclat du teint et les rides.** | |
|---|---|
| Daidzéine | 0,5 % |
| Hydroxypropylcellulose | 1,00 % |
| (Klucel H® vendu par la société Hercules) Antioxydant | 0,05 % |
| Isopropanol | 40,00 % |
| Conservateur | 0,30 % |
| Eau | qsp 100,00 % |

| **Composition 4 :** **Gel pour corriger le relâchement des tissus cutanés et sous- cutanés, notamment en régulant la synthèse et/ou la dégradation des protéines matricielles** | |
|---|---|
| Génistéine | 0,8 % |
| Hydroxypropylcellulose | 1,00 % |
| (Klucel H® vendu par la société Hercules) Antioxydant | 0,05 % |
| Chlorhydrate de lidocaïne | 2,00 % |
| Isopropanol | 40,00 % |
| Conservateur | 0,30 % |
| Eau | qsp 100,00 % |

| **Composition 5 :** **Crème de soin (émulsion huile/eau) pour réguler la séborrhée** | |
|---|---|
| 5 Hydroxyequol(4',5,7-trihydroxy-isoflavane) | 0,2 % |
| Stéarate de glycérol | 2,00 % |
| Polysorbate 60 (Tween 60® vendu par la société ICI) | 1,00 % |
| Acide stéarique | 1,40 % |
| Acide n-octanoyl-5-salicylique | 0,50 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100,00 % |

## Revendications

1. Composition cosmétique et/ou dermatologique à application topique ou par administration orale, **caractérisée en ce qu'**elle contient une quantité efficace d'au moins un composé inhibiteur de l'enzyme 3β-hydroxystéroïde déhydrogénase.

2. Composition selon la revendication 1, **caractérisée en ce que** le composé inhibiteur de l'enzyme 3β-hydroxystéroïde déhydrogénase est présent à raison de 0,0000001 à 25% en poids, et de préférence de 0,00001 à 10% en poids par rapport total de la composition.

3. Composition selon la revendication 1 ou 2, **caractérisée en ce que** le composé inhibiteur de l'enzyme 3β-hydroxystéroïde déhydrogénase est choisi parmi les isoflavones et ses dérivés, notamment isoflavanes.

4. Composition selon la revendication 3, **caractérisée en ce que** l'isoflavone est choisie parmi la daidzéine et la génistéine.

5. Composition selon la revendication 3, **caractérisée en ce que** le dérivé d'isoflavone est choisi parmi l'Equol et le 5-Hydroxy Equol.

6. Composition selon la revendication 1 ou 2, **caractérisée en ce que** le composé inhibiteur de l'enzyme 3β-hydroxystéroïde déhydrogénase est choisi parmi l'Epostane, le Trilostane, le 11-α-bromoacetoxy-progestérone, des dérivés 11-alkyl de la progestérone, des dérivés 3 ou 4 cétostéroïdes, le RU486 et le N,N-diméthyl-4-méthyl-3-oxo-4-aza-5-androstane-17-β-carboxamide (4-MA)

7. Composition selon la revendication 1 ou 2, caractérisée en que le composé inhibiteur de l'enzyme 3β-hydroxystéroïde déhydrogénase est choisi parmi le cholestérol, ses esters et ses dérivés, la 16,α-hydroxydehydroepiandrosterone, le 17-β-oestradiol, l'éthynylestradiol, l'acétate de chloramidone, le cyanotriméthylandrostenolone.

8. Composition selon l'une quelconque des revendications précédentes, **caractérisée en ce que** le composé inhibiteur de l'enzyme 3-β-hydroxystéroïde déhydrogénase est associé dans la composition à des hormones, naturelles ou synthétiques, choisies parmi les hormones oestrogéniques progestatives et androgéniques.

9. Composition selon la revendication 8, **caractérisée en ce que** les hormones sont choisies parmi la progestérone, l'oestradiol, la testostérone, le broparestrol, l'oestrone, l'acétate de prégnénolone, la prégnénolone, la 17-β-hydroxyprogestérone, le propionate de testostérone, l'androstène et les androstanédiols.

10. Composition selon l'une quelconque des revendications 1 à 6, **caractérisée en ce que** le composé inhibiteur de l'enzyme 3β-hydroxystéroïde déhydrogénase est associé dans la composition à des molécules et/ou des extraits végétaux mimétiques de l'oestradiol et/ou de la progestérone.

11. Composition selon l'une quelconque des revendications 1 à 6, **caractérisée en ce que** le composé inhibiteur de l'enzyme 3β-hydroxystéroïde déhydrogénase est associé dans la composition à de la DHEA et/ou du sulfate de DHEA et/ou de la 7OH-DHEA et/ou de la 7 Ceto-DHEA et/ou la 3β-acétoxy-7-oxo-DHEA.

12. Composition selon l'une quelconque des revendications précédentes, **caractérisée en ce que** la composition se présente sous forme d'une solution ou suspension aqueuse, alcoolique ou hydroalcoolique ou d'une suspension huileuse ou d'une solution ou d'une dispersion du type lotion ou sérum, d'une émulsion de consistance liquide ou semi-liquide du type lait, obtenues par dispersion d'une phase grasse dans une phase aqueuse (H/E) ou inversement (E/H), ou d'une suspension ou émulsion de consistance molle de type crème (H/E) ou (E/H), ou d'un gel aqueux ou anhydre, d'un onguent, d'une poudre libre ou compactée à utiliser telle quelle ou à incorporer dans un excipient, ou sous forme de microcapsules ou microparticules, ou de dispersions vésiculaires de type ionique et/ou non ionique.

13. Composition selon la revendication 12, **caractérisée en ce que** la composition comprend au moins une matière grasse liquide ou solide choisie parmi les huiles minérales, les huiles végétales, les huiles animales, les huiles de synthèse, les huiles ou les cires siliconées, les huiles fluorées, les cires d'abeille, de Carnauba, ou de paraffine, les acides gras, les alcools gras et les cires.

14. Composition selon l'une quelconque des revendications précédentes, **caractérisée en ce que** la composition contient un ou plusieurs adjuvants choisis parmi les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes.

15. Composition selon la revendication 14, **caractérisée en ce que** les gélifiants hydrophiles sont choisis parmi les polymères carboxyvinyliques, les copolymères acryliques, les polyacrylamides, les polysaccharides, les gommes naturelles et les argiles.

16. Composition selon la revendication 14, **caractérise en ce que** les actifs lipophiles ou hydrophiles sont choisis parmi les rétinoïdes, les α-hydroxy-acides, les β-hydroxy-acides, les α-céto-acides, les β-céto-acides, les peroxydes, les vitamines et de préférence les vitamines E et F, les actifs anti-radicaux, les sélénium, le zinc et les β-carotène.

17. Composition selon la revendication 14, **caractérisée en ce que** les gélifiants lipophiles sont choisis parmi les argiles modifiées, les sels métalliques d'acides gras, l'éthylcellulose et le polyéthylène.

18. Utilisation dans une composition cosmétique et/ou dermatologique à application topique, d'une quantité efficace d'au moins un composé inhibiteur de l'enzyme 3β-hydroxystéroïde déhydrogénase en tant qu'agent d'activation du renouvellement cellulaire et/ou d'amélioration de la densité des macromolécules fibreuses du derme.

19. Utilisation dans une composition cosmétique et/ou dermatologique à application topique, d'une quantité efficace d'au moins un composé inhibiteur de l'enzyme 3β-hydroxystéroïde déhydrogénase en tant qu'agent régulateur de la synthèse et/ou de la dégradation des protéines matricielles du derme.

20. Utilisation dans une composition cosmétique et/ou dermatologique à application topique, d'une quantité efficace d'au moins un composé inhibiteur de l'enzyme 3β-hydroxystéroïde déhydrogénase en tant qu'agent de correction du relâchement des tissus cutanés et sous-cutanés.

21. Utilisation dans une composition cosmétique et/ou dermatologique à application topique, d'une quantité efficace d'au moins un composé inhibiteur de l'enzyme 3β-hydroxystéroïde déhydrogénase en tant qu'agent ravivant l'éclat de la peau et/ou de réduction des rides et ridules de la peau.

22. Utilisation dans une composition cosmétique et/ou dermatologique à application topique, d'une quantité efficace d'au moins un composé inhibiteur de l'enzyme 3β-hydroxystéroïde déhydrogénase en tant qu'agent de régulation des processus de pigmentation de la peau et des phanères et/ou de la séborrhée et/ou de la croissance du cheveu et/ou des poils et/ou des ongles.
